# EUROPÄISCHE PATENTSCHRIFT

(11) **EP 0 892 623 B1**
(45) Veröffentlichungstag und Bekanntmachung des Hinweises auf die Patenterteilung: **05.06.2002**
(21) Anmeldenummer: 97905083.8
(22) Anmeldetag: 25.02.1997
(51) Int. Cl.: A61B 17/70

(54) **WIRBELSÄULENFIXATEUR**
SPINAL FIXING DEVICE
DISPOSITIF DE CONTENTION POUR LA COLONNE VERTEBRALE

(30) Priorität: 09.04.1996 DE 29606468 U
(43) Veröffentlichungstag der Anmeldung: 27.01.1999
(73) Patentinhaber: Waldemar Link (GmbH & Co.), 22339 Hamburg (DE)
(72) Erfinder: KELLER, Arnold, D-23863 Kayhude (DE)
(74) Vertreter: Glawe, Delfs, Moll & Partner
(86) Internationale Anmeldenummer: EP9700900
(87) Internationale Veröffentlichungsnummer: WO9737604

(56) Entgegenhaltungen:
- DE-A- 4 107 480
- FR-A- 2 624 720
- FR-A- 2 640 492
- FR-A- 2 706 761

## Beschreibung

Die Erfindung betrifft einen Fixateur für Wirbelkörper oder Knochenfragmente, der eine Stange umfaßt, an der mindestens eine Pedikelschraube zu befestigen ist. Deren U-förmiger Kopf bildet eine Aufnahme für die Stange. Die Stange wird in der Aufnahme durch eine Fixiereinrichtung gehalten, die einen U-förmigen Spannbügel umfaßt, dessen Schenkel formschlüssig mit dem Kopf der Pedikelschraube verbindbar sind und dessen Steg eine Fixierschraube enthält. Bei einem bekannten Wirbelsäulenfixateur (DE-C 41 07 480) wirkt die Fixierschraube unmittelbar auf die in der Aufnahme liegende Stange. Das hat den Nachteil, daß die wechselnden Kräfte, die zwischen Stange und Pedikelschraube auftreten, auch auf die Schraube wirken und diese lc ern können, Das gilt insbesondere dann, wenn die Richtung der Stange nicht genau lotrecht zur Längsrichtung der Schraube verläuft und daher bei Relativbewegungen schon bei unbewegter Schraube ein Zustand größerer Lockerheit eintreten kann.

Der Erfindung liegt daher die Aufgabe zugrunde, einen Fixateur der eingangs genannten Art zu schaffen, der größere Verbindungssicherheit zwischen Stange und Pedikelschraube und eine bessere gegenseitige Ausrichtung auf die lotrechte Stellung ermöglicht.

Die erfindungsgemäße Lösung besteht darin, daß die Fixierschraube nicht unmittelbar oder nicht ausschließlich unmittelbar auf die Stange einwirkt, sondern dazu benutzt wird, einen U-förmigen Fixierbügel mit seinen Schenkelenden seitlich des Spannbügels gegen die Stange zu spannen.

Die Schenkelenden des Fixierbügels sitzen in einem gewissen Abstand vom Zentrum der Fixiereinrichtung auf der Stange auf und vemögen deshalb ein beträchtliches Richtmoment auf diese auszuüben, falls sie bei Beginn des Fixiervorgangs nicht lotrecht gegenüber der Pedikelschraube liegt. Mit großer Sicherheit wird dadurch im Laufe des Fixiervorgangs die lotrechte Stellung erreicht. Diese wird auch unter wechselnden Kräften mit großer Sicherheit aufrechterhalten. Relativbewegungen zwischen Stange und Fixierschraube und innerhalb der Fixiereinrichtung finden praktisch nicht statt. Daher greifen auch an der Fixierschraube keine Karfte aus wechselnden Richtungen an, die zu ihrer Lockerung führen können. Vielmehr wirken in der Fixierschraube nahezu ausschließlich axiale Kräfte.

Obwohl somit die Gefahr der Lockerung der Fixierschraube wesentlich geringer ist als bei der bekannten Lösung, kann sie in einer vorteilhaften Ausführungsform der Erfindung mit einer Sicherungseinrichtung versehen sein, insbesondere mit einer Kontermutter. Jedoch können auch andere bekannte Sicherungseinrichtungen für diese Zwecke eingesetzt werden.

Bei der eingangs beschriebenen, bekannten Anordnung sind die formschlüssig zusammenwirkenden Mittel am Kopf der Pedikelschraube und am Spannbügel als ineinander eingreifende Rippen und Nuten ausgebildet, die lotrecht zur Richtung der Pedikelschraube und parallel zu der Stange verlaufen. Wenn während der Operation der Kopf der Pedikelschraube mit der Stange verbunden werden muß, haben diese Teile häufig nicht von vornherein die Stellung, die ihnen zugedacht ist; sie müssen erst mit einem gewissen Kraftaufwand aufeinander ausgerichtet werden. Unter diesen Umständen kann es schwierig sein, die Fixiereinrichtung genau so auszurichten, daß sie auf den Kopf der Pedikelschraube aufgeschoben werden kann. Die Erfindung erleichtert diesen Vorgang dadurch, daß sie ein Werkzeug zum richtungsgenauen Halten der Fixiereinrichtung schafft. Das Werkzeug, das mit einem großen Handgriff ausgerüstet ist, kann leicht in der gewünschten Weise ausgerichtet werden, wodurch dann auch die korrekte Ausrichtung der Fixiereinrichtung gewährleistet ist. Damit die Fixiereinrichtung gegenüber dem Werkzeug korrekt ausgerichtet ist, weist das Werkzeug erfindungsgemäß einen sattelförmigen Halteteil auf, der zwei einander gegenüberliegende, rippenförmige Vorsprünge aufweist, die unter die Ränder des Stegs des Fixierbügels fassen. Dadurch werden die Ränder des Fixierbügels auf die Richtung der Rippen und somit auch auf die Richtung des Werkzeugs ausgerichtet.

Damit sich die Fixiereinrichtung von dem Werkzeug nicht ungewollt lösen kann, ist die Anordnung so getroffen, daß die rippenförmigen Vorsprünge des Werkzeugs zwischen den Rändern des Fixierbügels und die diesen gegenüberliegende Oberfläche des -Spannbügels geklemmt werden, indem die Fixierschraube nach dem Einsetzen der Fixiereinrichtung angezogen wird. Dabei soll der durch die rippenförmigen Vorsprünge bestimmte Abstand zwischen dem Spannbügel und dem Fixierbügel größer sein als der Abstand zwischen Teilen im Fixierzustand. Der Fixierbügel ist somit gegenüber der Stange noch ein wenig angehoben, und es ist genügend Spiel vorhanden, um die Fixiereinrichtung auf den Kopf der Pedikelschraube zu schieben, auch wenn sich die Stange bereits in der Aufnahme der Pedikelschraube befindet. Diese Anordnung, bei der die Fixiereinrichtung an dem Werkzeug festgespannt wird, indem die Rippen des Werkzeugs zwischen Fixierbügel und Spannbügel eingeklemmt werden, hat den Vorteil, daß die Fixiereinrichtung an dem Werkzeug wackelfrei festsitzt. Das erleichtert das Aufsetzen auf den Kopf der Pedikelschraube. Es bedingt allerdings, daß das Werkzeug vor dem Festziehen der Fixiereinrichtung am Pedikelkopf entfernt wird, weil der Fixierzustand nicht erreichbar ist, solange sich die Fixiereinrichtung am Werkzeug befindet. In vielen Fällen stört dies nicht, weil der schwierigste Teil der Manipulation das Aufschieben der Fixiereinrichtung auf den Kopf der Pedikelschraube ist und das nachfolgende Festziehen keine besonderen Probleme bietet.

Jedoch kann das Verfahren vereinfacht werden, wenn die Halterung der Fixiereinrichtung an dem Werkzeug so gestaltet ist, daß die Fixiereinrichtung an der Pedikelschraube festgezogen werden kann, solange sie noch im Werkzeug gehalten ist. Zu diesem Zweck wird der im Fixierzustand bestehende Abstand zwischen den Stegen des Fixierbügels und des Spannbügels größer bemessen als die Höhe der die Fixiereinrichtung am Werkzeug haltenden Rippen. Das Werkzeug kann daher auch dann noch von der Fixiereinrichtung abgezogen werden, wenn diese an der Pedikelschraube festgespannt ist. Dabei nimmt man in Kauf, daß die Fixiereinrichtung nicht am Werkzeug durch Zusammenziehen der beiden Bügel verspannt werden kann. Jedoch kann eine Fixierung am Werkzeug dadurch erreicht werden, daß das Werkzeug und ein Schraubenzieher zum Betätigen der Fixierschraube derart verbunden sind, daß der am Schraubenkopf angesetzte Schraubenzieher gleichzeitig ein Hindernis für das Abziehen des Fixiereinrichtung von dem Werkzeug bildet.

In jedem Falle hat es sich als zweckmäßig erwiesen, wenn das Werkzeug einen Griffschaft besitzt, der als Führung für einen mit der Fixierschraube zusammenwirkenden Schraubenzieher ausgebildet ist. Das bloße Einschieben des Schraubenziehers in die Führung gewährleistet, daß der Schraubenzieher ohne weiteres die Stellung findet, in der er in die Fixierschraube eingreift. In dieser Stellung verriegelt er die Fixiereinrichtung gegen eine Bewegung, deren Richtung quer zur Richtung des Schraubenziehers verläuft und sichert sie dadurch am Werkzeug.

Nicht nur das Aufschieben der Fixiereinrichtung auf den Pedikelschraubenkopf kann sich unter Operationsbedingungen schwierig gestalten, sondern auch nach dem Aufschieben das Auffinden der korrekten Zentrierposition der Fixiereinrichtung gegenüber der Pedikelschraube. Erfindungsgemäß kann dies dadurch vereinfacht werden, daß der Kopf der Pedikelschraube und die Fixiereinrichtung ein zusammenwirkendes Anschlagpaar aufweisen, das die Position der Fixiereinrichtung in der korrekten Stellung gegenüber dem Kopf der Pedikelschraube in einer Richtung begrenzt. Beim Aufschieben der Fixiereinrichtung auf den Schraubenkopf ergibt sich dann die richtige Position von selbst.

Dasselbe Prinzip kann benutzt werden, um das Einsetzen der Fixiereinrichtung in das Werkzeug zu erleichtern.

Die Erfindung wird im folgenden näher unter Bezugnahme auf die Zeichnung erläutert, die ein vorteilhaftes Ausführungsbeispiel veranschaulicht. Darin zeigen:

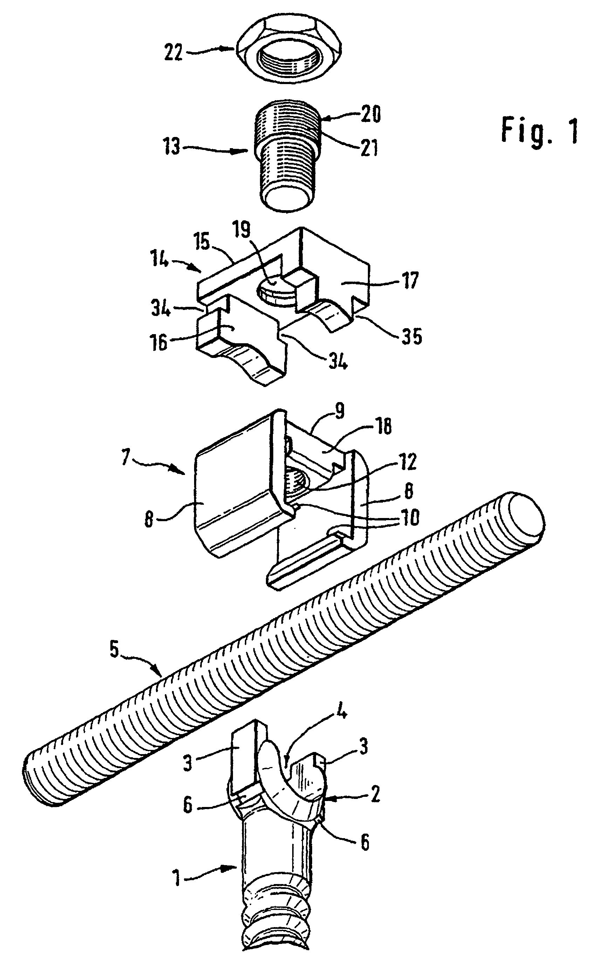

- Fig. 1: eine Explosionsansicht der Pedikelschraube, der Stange und der Fixiereinrichtung,
- Fig. 2: eine Ansicht in Stangenrichtung der Pedikelschraube und der in einem Werkzeug gehaltenen Fixiereinrichtung,
- Fig. 3: eine der Figur 2 entsprechende Seitenansicht aus der entgegengesetzten Richtung nach dem Verbinden mit der Pedikelschraube,

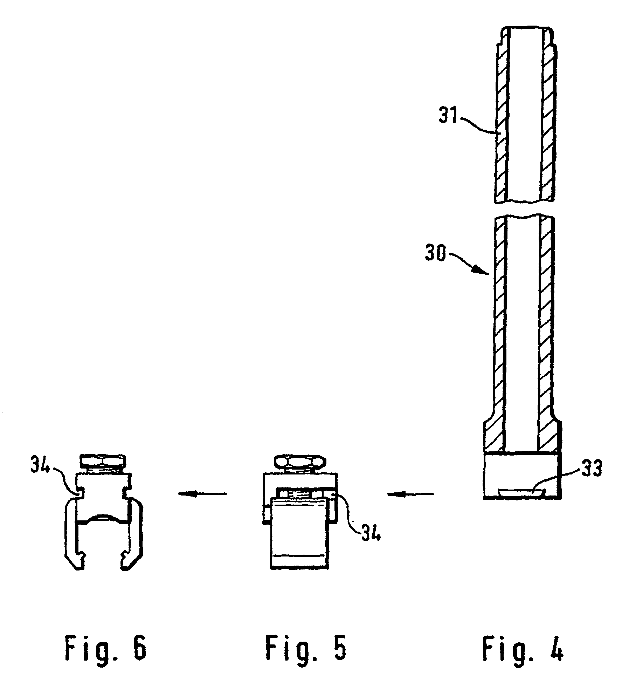

- Fig. 4: eine Schnittansicht des Werkzeugs,
- Fig. 5: und
- Fig. 6: zwei Seitenansichten der Fixiereinrichtung,

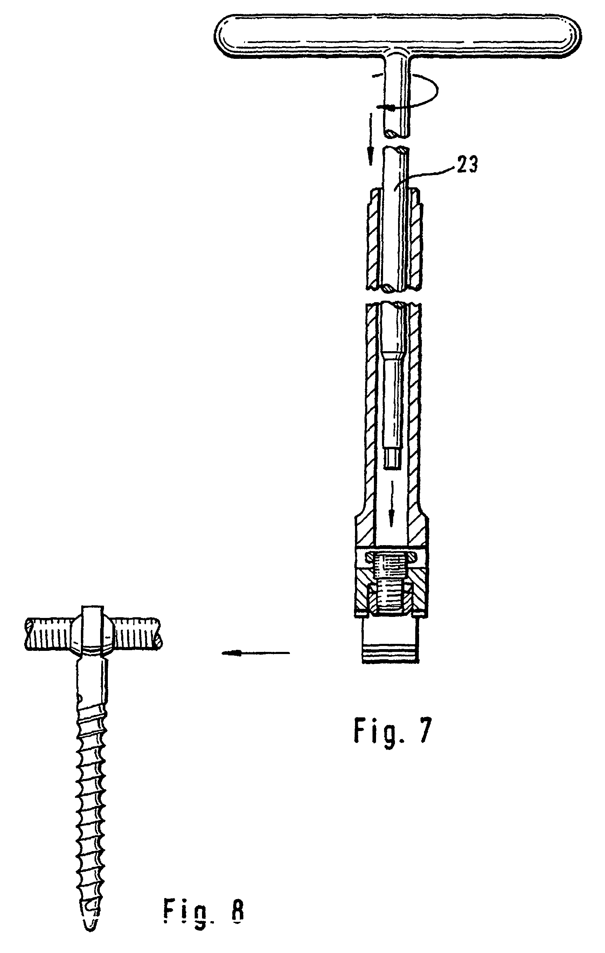

- Fig. 7: einen Schnitt durch das Werkzeug mit der darin gehaltenen Fixiereinrichtung und Schraubenzieher,
- Fig. 8: die Pedikelschraube mit einer in deren Aufnahme eingesetzter Stange, bevor die Fixiereinrichtung aufgesetzt wird,
- Fig. 9: diese Teile im zusammengesetzten Zustand und
- Fig. 10: die Pedikelschraube, die Stange und die Fixiereinrichtung im fertig montierten Zustand.

Die Pedikelschraube 1 weist einen Kopf 2 auf, der von zwei U-förmig gabeligen Schenkeln 3 gebildet wird, zwischen denen sich die Aufnahme 4 für die Stange 5 befindet. Nahe den schaftseitigen Enden der Schenkel 3 befinden sich Nuten 6, die lotrecht zur Richtung des Schraubenschafts und parallel zu der durch die Aufnahme 4 festgelegten Stangenrichtung verlaufen. Die Außenflächen der Schenkel 3 sind parallel zueinander und zu der durch die Schraubenlängsrichtung und die Stangenrichtung bestimmten Ebene.

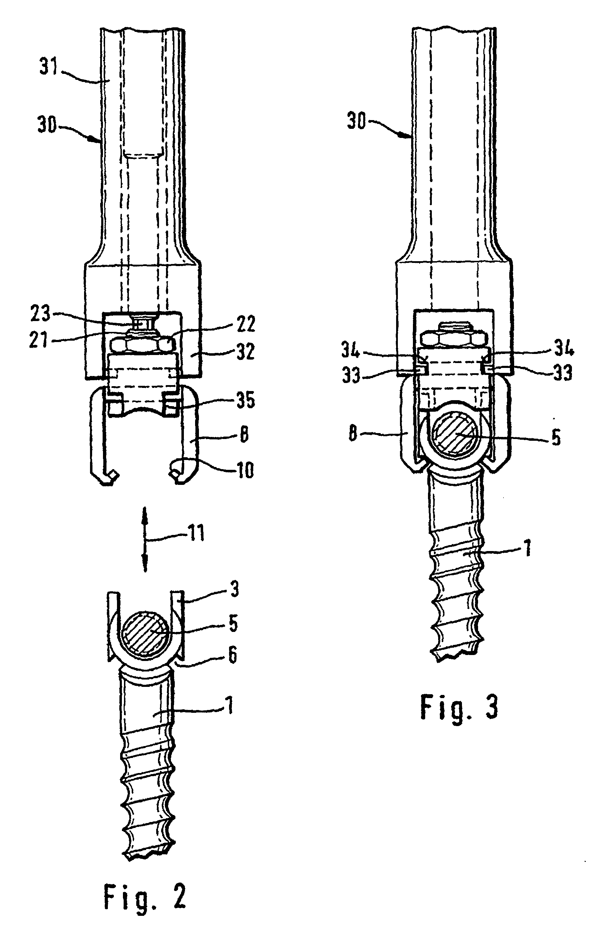

Der Spannbügel 7 besteht U-förmig aus zwei plattenförmigen, zueinander parallelen Schenkeln 8 und einem Steg 9. Die lichte Weite zwischen den Schenkeln 8 gleicht der Breite des Schraubenkopfs zwischen den Außenflächen der Schenkel 3. An ihren öffnungsseitigen Enden sind die Schenkel 8 zueinander hingebogen und tragen in diesem Bereich auf der Innenseite je eine Rippe 10, die passend zu den Nuten 6 des Pedikelschraubenkopfs gestaltet ist. Der Schraubenkopf kann daher in den zwischen den Schenkeln 8 und dem Steg 9 des Fixierbügels gebildeten Raums passend eingeschoben werden, wie dies in Fig.3 veranschaulicht ist. Man erkennt, daß die Flanken der Rippen 10 und der Nuten 6 derart geneigt verlaufen, daß sie die beiden Teile formschlüssig zusammenhalten, wenn sie durch im Sinne des Pfeils 11 (Fig.2) gerichtete Kräfte auseinandergezogen werden.

Der Steg 9 des Spannbügels 7 weist mittig eine Gewindebohrung 12 auf, zu der eine Fixierschraube 13 paß⁺. Diese dient zum Spannen des Fixierbügels 14 auf den Spannbügel 7. Der Fixierbügel 14 besteht aus einem Steg 15 und zwei Schenkeln 16, 17, deren Breite mit geringem Spiel der lichten Weite zwischen den Schenkeln 8 des Spannbügels 7 entspricht. Die Stirnflächen 18 des Stegs 9 des Spannbügels 7 sind gegenüber den Kanten der Schenkel 8 ein wenig zurückversetzt. Die lichte Weite zwischen den Schenkeln 16, 17 des Fixierbügels 14 ist geringer als die Breite der Schenkel 8 und größer als diejenige des Stegs 9 des Spannbügels 7. Wenn der Fixierbügel 14 auf den Spannbügel 7 aufgesetzt ist, werden daher die Schenkel 16, 17 des Fixierbügels zwischen den Schenkeln 8 des Spannbügels geführt. Der Steg 15 des Fixierbügels enthält in der Mitte eine Durchgangsbohrung 19 für die Fixierschraube 13, deren Kopf 20 sich auf die Oberseite des Stegs 15 oder in eine entsprechende Einsenkung desselben aufsetzt, wenn die Teile zusammengezogen werden. Der Kopf 20 der Fixierschraube 13 ist mit Außengewinde 21 passend zu einer Kontermutter 22 versehen, die zur Sicherung der Fixierschraube im montierten zustand dient. Die Fixierschraube 13 enthält im Kopf 20 eine Innensechskantausnehmung für den Angriff eines Schraubenziehers 23.

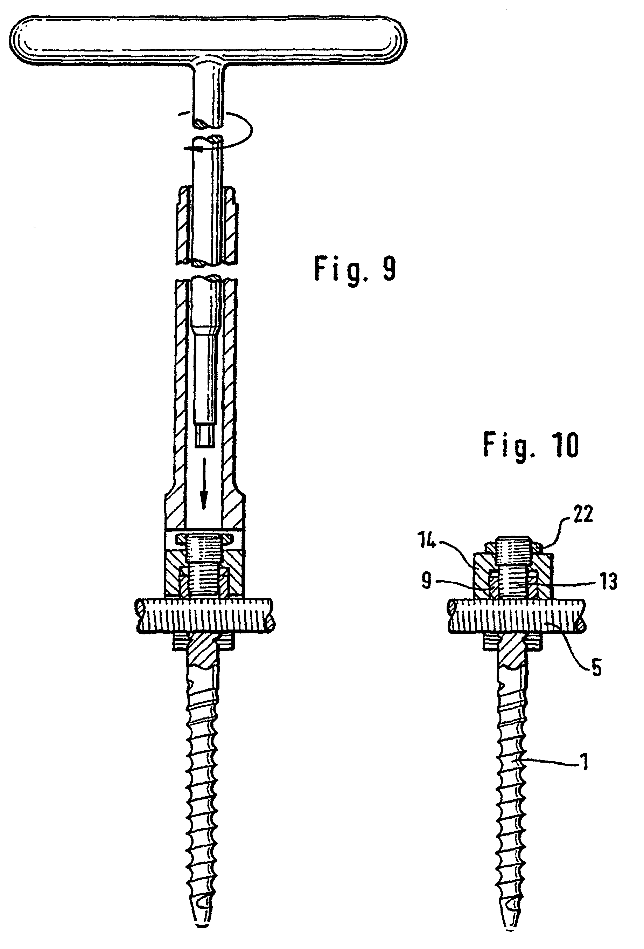

Wenn die in Fig.2 gezeigten Teile fertig zusammengesetzt sind, ergibt sich das in Fig.10 gezeigte Bild. Die Stange 5 liegt in der Aufnahme 4 der Pedikelschraube. Der Spannbügel 7, von dem in Fig.10 lediglich ein Teil des Stegs 9 sichtbar ist, ist über den Kopf der Pedikelschraube geschoben und mit diesem über die Rippen 10 und Nuten 6 formschlüssig verbunden. Mittels der Fixierschraube 13 ist der Fixierbügel 14 so verspannt, daß die Enden seiner Schenkel 16, 17 auf der Stange 5 aufsitzen. Sie wird dadurch festgehalten und rechtwinklig zur Pedikelschraube 1 ausgerichtet. Das Ende der Pixierschraube 13 kann dabei zusätzlich zu den Enden der Schenkel 16, 17 des Fixierbügels auf der Stange 5 aufsitzen; erwünscht ist dies aber normalerweise nicht. Durch die Kontermutter 12 ist die Fixierschraube 13 in ihrer Lage gesichert.

Um diese Anordnung zu erhalten, muß die aus den Bügeln 7, 14 der Schraube 13 und Kontermutter 22 bestehende Fixiereinrichtung über den Kopf der Pedikelschraube 1 geschoben werden, nachdem die Stange 5 in der Aufnahme 4 ihren Platz gefunden hat. Dies geschieht mittels des Werkzeugs 30, das eine kräftige und bequem handhabbare Griffstange 31 aufweist und an deren Ende eine sattel- oder gabelförmige Halterung 32 für die Fixiereinrichtung bildet. Die Halterung 32 hat eine lichte Weite, die der Breite des Stegs 15 des Fixierbügels 14 mit Spiel gleicht und weist am unteren Ende zwei einander gegenüberstehende Rippen 33 auf, mit denen es unter die Ränder des Stegs 15 der Fixierplatte geschoben wird. Damit dies möglich ist, weist der Schenkel 16 benachbart zum Steg 15 Ausschnitte 34 auf, die der Querschnittsgröße der Rippen 33 entsprechen. Der Steg 17 enthält solche Ausschnitte nicht. Wird das Werkzeug mit seiner Halterung 32 auf den Steg 15 des Fixierbügels aufgeschoben, wird seine Endposition daher durch den Anschlag der Enden der Rippen 33 am Schenkel 17 bestimmt. Diese Teile bilden einen Anschlag der die sichere Positionierung der Fixiereinrichtung in der Halterung 32 des Werkzeugs 30 bestimmt. In dieser Position kann die Fixiereinrichtung am Werkzeug in unten näher beschriebener Weise verriegelt werden.

Werkzeug und Fixiereinrichtung bilden dann eine bequem gemeinsam handhabbare Einheit, wobei die Fixiereinrichtung gegenüber dem Kopf 2 der Pedikelschraube 1, auf den sie aufgesetzt werden soll, mittels des Workzeugs leicht gefunden werden kann. Es fällt auch nicht schwer, mittels des Werkzeugs und über die Fixiereinrichtung auf die Stange Druck auszuüben, falls diese sich gegen die Einfügung in die Aufnahme 4 der Pedikelschraube sperrt.

Damit die Fixiereinrichtung auf den Kopf 2 der Pedikelschraube aufgesetzt werden kann, obwohl die Schenkel 3 der Pedikelschraube sich über die gesamte innere Höhe des Spannbügels 7 erstrecken, weist der Schenkel 17 des Fixierbügels Ausschnitte 35 auf. Der andere Schenkel 16 besitzt solche Ausschnitte nicht. Die Fixiereinrichtung kann daher nur von einer Seite her und in einer Richtung auf die Pedikelschraube aufgesetzt werden. Der Sinn dieser Anordnung besteht darin, daß durch den Schenkel 16 im Zusammenwirken mit dem Schraubenkopf 2 ein Anschlag gebildet wird, durch den die richtige Endstellung der Fixiereinrichtung gegenüber dem Schraubenkopf bestimmt wird.

Wenn die Fixiereinrichtung diese Position erreicht hat, werden die Fixierschraube 13 und die Kontermutter 22 festgezogen.

Es gibt zwei Möglichkeiten, die Fixiereinrichtung an dem Werkzeug 30 während der Montage sicher festzuhalten. Die erste dieser Möglichkeiten ist in Fig.3 demonstriert. Die Stege 15 und 9 der Fixier- und Spannbügel 14 bzw. 7 sind mittels der Schraube 13 so zusammengezogen, daß die Rippen 33 des Werkzeugs 30 eingeklemmt werden zwischen der Unterseite der Ränder des Steg 15 und der Oberseite des Stegs 9. Diese Methode der Sicherung hat den Vorteil, daß sämtliche Teile der Fixiereinrichtung starr mit dem Werkzeug verbunden sind und der Arzt daher ein sehr sicheres Gefühl für die Lage der Fixiereinrichtung bei der Montage hat. Voraussetzung dafür ist, daß in dem in Fig.3 gezeigten Zustand, wenn also die Rippen 33 zwischen den Bügeln eingeklemmt sind, noch hinreichendes Spiel unter den Enden der Schenkel 16, 17 gegenüber der Stange 5 vorhanden ist. Andernfalls könnte die Fixiereinrichtung nicht auf die Pedikelschraube und die darin befindliche Stange 5 aufgeschoben werden. Dies hat den Nachteil, daß der Spannbügel 14 nicht gegen die Stange 5 gespannt werden kann, solange das Werkzeug 30 mit der Fixiereinrichtung verbunden ist. Diese muß vielmehr erst abgenommen werden, bevor die Fixierschraube 13 angezogen wird.

Die andere Möglichkeit zur Verriegelung der Fixiereinrichtung am Werkzeug 30 wird durch Fig.2 demonstriert. Wie zuvor beschrieben, ist das Werkzeug so mit der Fixiereinrichtung verbunden, daß die Rippen 33 unter den Rändern des Stegs 15 des Fixierbügels 14 liegen. An der Entfernung aus dieser Lage wird die Fixiereinrichtung dadurch gehindert, daß der Schraubenzieher 23, der durch eine Bohrung des Griffs 31 des Werkzeugs 30 geführt ist, in die Sechskantvertiefung des Schraubenkopfs 21 eingreift. Diese Verriegelungsposition des Schraubenziehers 21 kann dadurch aufrechterhalten werden, daß entweder die Griffe des Schraubenziehers und des Werkzeugs gemeinsam gehalten werden und dadurch eine entsprechende Kraft auf den Schraubenzieher ausgeübt wird oder daß zwischen diesen Teilen eine Federanordnung wirkt, die den Schraubenzieher 23 in Fig.2 nach unten drückt. Bei dieser Art der Sicherung der Fixiereinrichtung am Werkzeug verzichtet man darauf, die Rippen 33 des Werkzeugs zwischen den beiden Bügeln einzuklemmen, und kann daher den Abstand zwischen den Stegen 9 und 15 der Bügel so groß gestalten, daß die Rippen 33 auch dann noch nicht festgeklemmt werden; wenn der Fixierbügel 14 gegen die Stange 5 gespannt wird. Dieser Vorgang kann daher erfolgen, solange das Werkzeug 30 noch mit der Fixiereinrichtung verbunden ist. Dies hat nicht nur den Vorteil, daß ein Arbeitsschritt entfällt, sondern erleichtert auch das Auffinden der Fixierschraube 13 mit dem Schraubenzieher 23 in dem mitunter unübersichtlichen Operationsfeld. Der Schraubenzieher wird nämlich durch die Bohrung im Werkzeug 30 zwangsläufig zum Schraubenkopf 21 geführt und befindet sich danach ständig im Eingriff mit diesem.

## Patentansprüche

1. Fixateur für Wirbel- oder Knochenfragmente mit einer Stange (5) und mindestens einer daran zu befestigenden Pedikelschraube (1), deren U-förmiger Kopf (2) eine Aufnahme (4) für die Stange (5) bildet, und einer Pixiereinrichtung (7,14,13) zum fixieren der Stange (5) in der Aufnahme (4), die einen U-förmigen Spannbügel (7) umfaßt, dessen Schenkel (8) formschlüssig mit dem Kopf (2) der Pedikelschraube (1) verbindbar sind und dessen Steg (9) eine Fixierschraube (13) enthält, **dadurch gekennzeichnet, daß** die Fixiereinrichtung einen U-förmigen Fixierbügel (14) mit Seitlichen Schenkeln(16, 17) aufweist, wobei mittels der Fixierschraube (13) der U-förmige Fixierbügel (14) mit den Enden seiner Schenkel (16,17) seitlich des Spannbügels (7) gegen die Stange (5) spannbar ist.

2. Fixateur nach Anspruch 1, **dadurch gekennzeichnet, daß** die Fixierschraube (13) mit einer Sicherungseinrichtung (22) versehen ist.

3. Fixateur nach Anspruch 1 oder 2, **dadurch gekennzeichnet, daß** die formschlüssig zusammenwirkenden Mittel (6,10) am Kopf (2) die Pedikelschraube (1) und am Spannbügel (7) so ausgebildet sind, daß der Spannbügel (7) (bezogen auf die Längsrichtung der Pedikelschraube (1)) von der Seite her auf den Kopf (2) der Pedikelschraube (1) aufschiebbar ist und daß ein Werkzeug (30) zum Halten der Fixiereinrichtung vorgesehen ist, dessen Halteteil (32) zwei einander gegenüberliegende rippenförmige Vorsprünge (33) aufweist, die unter die Ränder des Stegs (15) des Fixierbügels fassen.

4. Fixateur nach Anspruch 3, **dadurch gekennzeichnet, daß** die rippenförmigen Vorsprünge (33) mittels der Fixierschraube (13) zwischen dem Fixierbügel (14) und dem Spannbügel (7) klemmbar sind.

5. Fixateur nach Anspruch 4, **dadurch gekennzeichnet, daß** der durch die rippenförmigen Vorsprünge (33) bestimmte Abstand zwischen dem Spannbügel (7) und dem Fixierbügel (14) größer ist als der Abstand dieser Teile im Fixierzustand.

6. Fixateur nach Anspruch 4, **dadurch gekennzeichnet, daß** der im Fixierzustand bestehende Abstand zwischen den Stegen (9,15) des Spannbügels (7) und des Fixierbügels (14) größer ist als die Höhe der Rippen (33).

7. Fixateur nach einem der Ansprüche 4 bis 6, **dadurch gekennzeichnet, daß** das Werkzeug (30) einen Griffschaft (31) besitzt, der als Führung für einen mit der Fixierschraube (13) zusammenwirkenden Schraubenzieher (23) ausgebildet ist.

8. Fixateur nach einem der Ansprüche 1 bis 7, **dadurch gekennzeichnet, daß** die Fixiereinrichtung und der Kopf (2) der Pedikelschraube (1) ein zusammenwirkendes Anschlagpaar (3,16) aufweisen, das die Fixierposition der Fixiereinrichtung gegenüber dem Kopf (2) der Pedikelschraube (1) in einer Richtung begrenzt.

9. Fixateur nach einem der Ansprüche 3 bis 8, **dadurch gekennzeichnet**, das die Fixiereinrichtung und der Halteteil (32) des Werkzeugs (30) ein zusammenwirkendes Anschlagpaar (33,17) aufweisen, welches die Halteposition des Werkzeugs (30) gegenüber der Fixiereinrichtung begrenzt.

## Claims

1. A fixator for vertebral or bone fragments having a rod (5) and at least one pedicle screw (1) to be fastened thereto, the U-shaped head (2) of which screw forms a socket (4) for the rod (5), and having a locating device (7,14,13) for locating the rod (5) in the socket (4) and which comprises a U-shaped clamp (7), the arms (8) of which can be connected in a form-locking manner with the head (2) of the pedicle screw (1) and the cross-piece (9) of which includes a locating screw (13), **characterised in that** the locating device has a U-shaped locating clip (14) with lateral arms (16,17), wherein by means of the locating screw (13) the U-shaped locating clip (14), with the ends of its arms (16,17) laterally of the clamp (7), can be tightened against the rod (5).

2. A fixator according to Claim 1, **characterised in that** the locating screw (13) is provided with a locking means (22).

3. A fixator according to Claim 1 or 2, **characterised in that** the means (6,10) co-operate in a form-locking manner on the head (2), the pedicle screw (1) and the clamp (7) are so formed that the clamp (7) (in relation to the longitudinal direction of the pedicle screw (1)) can be pushed from the side on to the head (2) of the pedicle screw (1), and **in that** a tool (30) is provided for retaining the locating device, the retaining part (32) of which has two mutually opposed rib-like projections (33) which engage under the edges of the cross-piece (15) of the locating clip.

4. A fixator according to Claim 3, **characterised in that** the rib-like projections (33) can be clamped by means of the locating screw (13) between the locating clip (14) and the clamp (7).

5. A fixator according to Claim 4, **characterised in that** the distance between the clamp (7) and the locating clip (14) determined by the rib-like projections (33) is greater than the distance apart of these parts in the locating position.

6. A fixator according to Claim 4, **characterised in that** the distance present in the locating position between the cross-pieces (9,15) of the clamp (7) and of the locating clip (14) is greater than the height of the ribs (33).

7. A fixator according to any one of Claims 4 to 6, **characterised in that** the tool has a grip shank (31) which is in the form of a guide for a screwdriver (23) co-operating with the locating screw (13).

8. A fixator according to any one of Claims 1 to 7, **characterised in that** the locating device and the head (20) of the pedicle screw (1) have a co-operating pair of stops (3,16) which limit the locating position of the locating device with respect to the head (2) of the pedicle screw (1) in one direction.

9. A fixator according to any one of Claims 3 to 8, **characterised in that** the locating device and the retaining part (32) of the tool (30) have a co-operating pair of stops (33,17) which limit the retaining position of the tool (30) with respect to the locating device.

## Revendications

1. Appareil de fixation pour des fragments de vertèbres ou d'os avec une tige (5) et au moins une vis pédiculée (1) à fixer sur celle-ci, dont la tête en forme de U (2) forme un réceptacle (4) pour la tige (5) et avec un dispositif de fixation (7, 14, 13) pour la fixation de la tige (5) dans le réceptacle (4), qui comprend un étrier de serrage en forme de U (7), dont les branches (8) peuvent être reliées en complémentarité de forme avec la tête (2) de la vis pédiculée (1) et dont la nervure (9) comprend une vis de fixation (13), **caractérisé en ce que** le dispositif de fixation comprend un étrier de fixation en forme de U (14) avec des branches latérales (16, 17), les extrémités des branches (16, 17) de l'étrier de fixation en forme de U (14) pouvant être serrées sur le côté de l'étrier de serrage (7) contre la tige (5) à l'aide de la vis de fixation (13).

2. Appareil de fixation selon la revendication 1, **caractérisé en ce que** la vis de fixation (13) est munie d'un dispositif de blocage (22).

3. Appareil de fixation selon la revendication 1 ou 2, **caractérisé en ce que** les éléments agissant ensemble en complémentarité de forme (6, 10) sont conçus de telle manière à la tête (2) de la vis pédiculée (1) et à l'étrier de serrage (7) que l'étrier de serrage (7) puisse être enfilé par le côté (par rapport à la direction longitudinale de la vis pédiculée (1) sur la tête (2) de la vis pédiculée (1) et **en ce qu'**il est prévu un outil (30) pour le maintien du dispositif de fixation, dont la partie de maintien (32) comprend deux avancées en forme de nervures (33) opposées l'une à l'autre, qui se positionnent sous les bords de la nervure (15) de la bride de fixation.

4. Appareil de fixation selon la revendication 3, **caractérisé en ce que** les avancées en forme de nervures (33) peuvent être serrées entre l'étrier de fixation (14) et l'étrier de serrage (7) au moyen de la vis de fixation (13).

5. Appareil de fixation selon la revendication 4, **caractérisé en ce que** l'espacement entre l'étrier de serrage (7) et l'étrier de fixation (14) déterminé par les avancées en forme de nervures (33) est plus grand que l'espacement de ces pièces en état de fixation.

6. Appareil de fixation selon la revendication 4, **caractérisé en ce que** l'espacement existant en état de fixation entre les nervures (9, 15) de l'étrier de serrage (7) et de l'étrier de fixation (14) est plus important que la hauteur des nervures (33).

7. Appareil de fixation selon l'une des revendications 4 à 6, **caractérisé en ce que** l'outil (30) comprend une tige de préhension (31), qui est conformée comme guide pour un tournevis (23) agissant de concert avec la vis de fixation (13).

8. Appareil de fixation selon l'une des revendications 1 à 7, **caractérisé en ce que** le dispositif de fixation et la tête (2) de la vis pédiculée (1) comprennent une paire de butées (3, 16), qui délimite la position de fixation du dispositif de fixation par rapport à la tête (2) de la vis pédiculée (1) dans une direction.

9. Appareil de fixation selon l'une des revendications 3 à 8, **caractérisé en ce que** le dispositif de fixation et la partie de maintien (32) de l'outil (30) comprennent une paire de butées agissant de concert (33, 17), qui délimite la position de maintien de l'outil (30) par rapport au dispositif de fixation.
